# EUROPÄISCHE PATENTSCHRIFT

(11) **EP 2 983 576 B1**
(45) Veröffentlichungstag und Bekanntmachung des Hinweises auf die Patenterteilung: **01.02.2017**
(21) Anmeldenummer: 14716300.0
(22) Anmeldetag: 08.04.2014
(51) Int. Cl.: A61B 3/00, A61B 3/12, A61B 3/15

(54) **VERFAHREN ZUR FOTOGRAFISCHEN BEOBACHTUNG UND/ODER DOKUMENTATION DES FUNDUS EINES AUGES SOWIE FUNDUSKAMERA**
METHOD FOR PHOTOGRAPHICALLY OBSERVING AND/OR DOCUMENTING THE FUNDUS OF AN EYE, AND FUNDUS CAMERA
PROCÉDÉ D'OBSERVATION ET/OU DOCUMENTATION PHOTOGRAPHIQUE DU FOND D'UN IL AINSI QUE CAMÉRA DE FOND D'OEIL

(30) Priorität: 08.04.2013 DE 102013005869
(43) Veröffentlichungstag der Anmeldung: 17.02.2016
(73) Patentinhaber: Voigtmann GmbH, 90443 Nürnberg (DE); Talkingeyes & More GmbH, 91052 Erlangen (DE); Friedrich-Alexander-Universität Erlangen-Nürnberg, 91054 Erlangen (DE); Universitätsklinikum Erlangen, 91054 Erlangen (DE)
(72) Erfinder: MICHELSON, Georg, 91083 Baiersdorf (DE); HÖHER, Bernhard, 91054 Erlangen (DE); SCHMAUSS, Bernhard, 92637 Theisseil (DE); VOIGTMANN, Peter, 90607 Rückersdorf (DE); KÖHLER, Thomas, 90419 Nürnberg (DE)
(74) Vertreter: Stippl, Hubert
(86) Internationale Anmeldenummer: PCT/EP2014/057069
(87) Internationale Veröffentlichungsnummer: WO 2014/166954

(56) Entgegenhaltungen:
- WO-A1-2005/020804
- WO-A1-2012/059236
- US-A- 5 570 157
- US-A1- 2005 110 951

## Beschreibung

Die vorliegende Erfindung betrifft ein Verfahren zur fotografischen Beobachtung und/oder Dokumentation des Fundus eines Auges sowie eine Funduskamera zur Erzeugung solcher Aufnahmen vorzugsweise ohne die Verwendung eines Mydriatikums d. h. eines die Pupille erweiternden Medikaments. Des Weiteren betrifft die vorliegende Erfindung ein Patientenführungsmodul für den Einsatz in einer Funduskamera.

### Technologischer Hintergrund

Mit einer Funduskamera, auch "Netzhautkamera" oder "Ophthalmoskop" genannt, werden fotografische Aufnahmen des Fundus (Augenhintergrund) angefertigt. Derartige Aufnahmen unterstützen die augenheilkundige Diagnostik. Sie dienen dazu, krankhafte Veränderungen der Netzhaut sichtbar zu machen und zu dokumentieren. Eine Funduskamera ist mit einer Beleuchtungsquelle, einer Beleuchtungsoptik sowie einer digitalen Kamera ausgestattet, die die Herstellung hochauflösender Aufnahmen ermöglicht, welche in einer digitalen Patientenakte gespeichert oder in ausgedruckter Form in der Patientenkarteikarte hinterlegt werden können.

Aufgrund der Beleuchtung kommt es bei einer Funduskamera zu Artefakten aufgrund von Rückreflexionen der Beleuchtungsquelle an verschiedenen Stellen im Auge. Diese Rückreflexionen werden auch Purkinjereflexionen genannt. Die Lage, Ausbildung und Entstehung dieser Purkinjereflexionen sind aus der US 4 729 652, auf deren Inhalt vollinhaltlich Bezug genommen wird, bekannt. Die erste, zweite und vierte Purkinjereflexion liegen in den Punkten ihrer scharfen Abbildung jeweils in etwa im Bereich der Linse des Auges. Die dritte Purkinjereflexion liegt im Punkt ihrer scharfen Abbildung in etwa zwischen der Linse und dem Fundus.

Die derzeit auf dem Markt befindlichen Funduskameras lassen sich je nach Methode der Artefaktunterdrückung in folgende verschiedene Gerätegruppen einteilen.

Bei der ersten Gerätegruppe wird eine ringförmige Pupillenteilung verwendet. Während die Pupille durch einen äußeren Beleuchtungsring beleuchtet wird, erfolgt die Detektion des vom Fundus zurückgestreuten Lichts durch eine kreisförmige, vom Belichtungslicht freie Mittelzone der Augenpupille. Zur Abgrenzung des äußeren Beleuchtungsrings vom inneren Detektionsbereich der Augenpupille ist zwischen den beiden Bereichen eine schmale Übergangszone vorgesehen, in der weder beleuchtet noch detektiert wird. Diese Übergangszone ist zweckmäßig, da hierdurch eine vollständige Trennung von Beleuchtungs- und Detektionsstrahlen nicht nur in der Corneaebene sondern in der gesamten Augenvorderkammer, d. h. von der Frontseite der Cornea bis zur Rückseite der Augenlinse erreicht werden kann. Mit solchen Funduskameras lassen sich zwar aufgrund der Trennung von Beleuchtung und Detektion von Purkinjereflexionen freie Bilder vom Augenhintergrund aufzeichnen, allerdings ist durch die ringförmige Pupillenteilung der erreichbare Funduswinkel vom Durchmesser der Pupille abhängig. Bei einem non-mydriatischen Auge, d. h. bei einem Auge, das nicht mit einem pupillenerweiterten Medikament (Mydriatikum) vorher behandelt worden ist, ist das Detektionsfeld sehr begrenzt.

Weiterhin gibt es Verfahren, bei denen polarisiertes Licht eingesetzt wird, um Rückreflexionen, vor allem solche Rückreflexionen, die innerhalb der Optik der Funduskamera entstehen, zu vermeiden bzw. zu reduzieren.

Ferner gibt es sogenannte Linien-Scanning-Verfahren, bei denen auf den Fundus eine leuchtende Linie produziert wird, die senkrecht zu ihrer Ausrichtung über den Fundus bewegt (gescannt) wird. Während dieses Vorgangs wird der Fundus auf eine digitale Kamera abgebildet, sodass ein komplettes Fundusbild belichtet wird.

### Nächstliegender Stand der Technik

Aus der WO 2012/059236 A1 ist bereits eine Funduskamera mit streifenförmiger Pupillenteilung sowie ein Verfahren zur Aufzeichnung artefaktfreier, hoch aufgelöster Fundusaufnahmen bekannt. Hierbei wird eine streifenförmige Pupillenteilung des zu untersuchenden Auges in Form eines senkrechten, die Beleuchtungszone kennzeichnenden Balkens sowie beidseitige, die Detektionszonen der Pupille darstellenden Kreissegmente vorgenommen. Darüber hinaus wird mittels einer verschiebbar ausgebildeten Spaltblende der Beleuchtungsquelle die Belichtung auf eine Spaltform begrenzt und scannend über den Augenhintergrund geführt. Das von der Netzhaut reflektierte Licht fällt als Abbild des Spaltes auf die betreffenden Sektoren eines ortsauflösenden Detektors und kann dort ausgelesen werden. Purkinjereflexionen werden bei dieser Methode mit aufgezeichnet. Um diese Reflexionen auf fertigen Aufnahmen zu vermeiden, wird von jedem aufgenommenen Hellbild ein zweites Bild (Dunkelbild) aufgenommen und zur Beseitigung der störenden Reflexionen vom Hellbild abgezogen.

### Aufgabe der vorliegenden Erfindung

Die Aufgabe der vorliegenden Erfindung besteht darin, ein Verfahren zur fotografischen Beobachtung, Dokumentation und/oder Diagnose der eingangsbeschriebenen Art zur Verfügung zu stellen, mittels dem einerseits ohne Verabreichung eines Mydriatikums ein sehr großer Bereich der Netzhaut aufgenommen werden kann, andererseits der Einsatz einer besonders kostengünstigen Variante einer Funduskamera ermöglicht wird und möglichst wenig Licht in das Auge des Untersuchten eingestrahlt wird, um die Lichtbelastung für den Untersuchten zu minimieren.

### Lösung der Aufgabe

Die vorstehende Aufgabe wird bei dem gattungsgemäßen Verfahren durch die Merkmale des Verfahrens gemäß Anspruch 1 gelöst. In Bezug auf die Funduskamera wird die Aufgabe durch eine Funduskamera gemäß den Merkmalen des Anspruchs 17 gelöst.

Zweckmäßige Ausgestaltungen der Erfindung werden in den abhängigen Ansprüchen beansprucht.

Das erfindungsgemäße Verfahren nach Anspruch 1 ermöglicht die Aufnahme von streifenförmigen Bereichen der Netzhaut, verbunden mit der Möglichkeit einer sehr einfachen und daher kostengünstigen apparativen Konzeption. Das Verfahren eröffnet ferner die Möglichkeit, entlang der Längsachse des Streifens einen sehr großen Bereich der Netzhaut aufzunehmen. Durch die Möglichkeit des Verzichts auf die Verabreichung eines Mydriatikums wird ein erhöhter Patientenkomfort geschaffen.

Dadurch, dass der detektierbare Beobachtungsbereich sowie nicht detektierbare Beleuchtungsbereich halbkreisförmig oder kreisabschnittförmige Form besitzen, kann insbesondere in Verbindung mit einer annähernd punktförmigen oder zumindest flächenmäßig begrenzten Beleuchtungsquelle mit einer hohen Strahlleistung pro Fläche die dritte und/oder vierte Purkinjereflexion vorteilhaft in ungenutzte Bereiche abgebildet werden.

Sofern die Aperturebene A1 von der Pupille nach hinten ins Auge hinein verlegt wird, vorzugsweise in eine Ebene an der Vorderseite der Linse des Auges oder zumindest in eine Ebene im Bereich der Linse des Auges, wird die vordere Linsenoberfläche von einem konvergenten Strahlenbündel durchsetzt und die hintere Linsenfläche von einem divergenten Strahlenbündel. Durch die entgegengesetzte Krümmung der Oberflächen der Linse des Auges kommt es bei der Reflexion der Beleuchtungsstrahlen jeweils zu einer Parallelisierung der reflektierten Strahlenbündel. Damit wird die Aperturebene A1 nach unendlich abgebildet, so dass sie genauso wie auch das Fundusbild die Fokuslage "unendlich" aufweist. Dies führt zu einer zumindest im Wesentlichen scharfen Abbildung der Aperturebene A1 und damit auch der Lichtquelle auf dem lichtempfindlichen elektronischen Bauelement der digitalen Kamera. Hierdurch werden Artefakte, wie z. B. die dritte und/oder vierte Purkinjereflexion auf besonders kleine Flächenbereiche im Fundusbild reduziert und können daher in den Randbereichen der streifenförmigen Dreiteilung der Bildfläche untergebracht werden.

Der streifenförmig beleuchtete Bereich der Bildfläche entspricht bei dem erfindungsgemäßen Verfahren einem Bildwinkel in Querrichtung zum Streifen von maximal 5° bis 30°, vorzugsweise von 18° bis 24°, besonders vorzugsweise von 20° bis 22°.

Ebenso entspricht der streifenförmig beleuchtete Bereich der Bildfläche einem Bildwinkel in Längsrichtung zum Streifen von 40° bis 90°, vorzugsweise 64° bis 72°, besonders vorzugsweise 66° bis 70° auf. Das Verfahren ermöglicht demzufolge, einen sehr großen Bereich der Netzhaut abzubilden.

Zweckmäßigerweise ist der Beleuchtungsstrahlengang und/oder der Detektionsstrahlengang während einer Aufnahme relativ zum Gesamtaufbau der Funduskamera ortsfest, d. h. keiner veränderbaren Translationsbewegung relativ zueinander und relativ zum Auge ausgesetzt. Aufwendige kostenintensive mechanische Komponenten können damit entfallen.

Dadurch, dass die im Beleuchtungsstrahlengang sowie Detektionsstrahlengang zu untersuchende Fläche des Fundus durch Veränderung der Blickrichtung des Auges des Patienten relativ zur Ausrichtung des Beleuchtungsstrahlengangs und/oder Detektionsstrahlengangs variiert wird, können auf diese Weise bei einem ortsfesten Beleuchtungsstrahlengang und/oder Detektionsstrahlengang dennoch Bereiche des Fundus, die über den beleuchteten Streifen hinausgehen, abgebildet werden und vorzugsweise zu einem Gesamtbild zusammengefügt werden. Hierdurch kann der zu untersuchende Bereich des Fundus erheblich vergrößert werden. Ebenso ist es möglich, den Bildwinkel des beleuchteten Bereichs der Bildfläche in Querrichtung zum Streifen zu reduzieren, beispielsweise auf weniger als 20°, vorzugsweise auf weniger als 18°, besonders vorzugsweise auf weniger als 16°, um Streueinflüsse der Lichtquelle weiter zu reduzieren.

Die "Führung" des menschlichen Auges erfolgt zweckmäßigerweise unter Erzeugung einer Fixationsmarke, die in ihrer Position verändert werden kann und auf dem Fundus abgebildet wird und deshalb gesehen wird.

Optional kann die Fixationsmarke von der Kamera aufgenommen und dokumentiert werden.

Zweckmäßigerweise wird bei dem erfindungsgemäßen Verfahren für jedes Auge getrennt eine Fixationsmarke erzeugt und zur Patientenführung verwendet. Im Gegensatz zu monokularen Verfahren wird hierbei die häufig zu beobachtende "Instrumentenakkommodation", also eine Störung der automatischen Entfernungsregelung im Gehirn, vermieden. Zudem ermöglicht diese Methode eine einzellfallbezogene Variation der Akkommodation und/oder der Konvergenz. Ohne Einsatz von beweglichen Teilen kann durch das erfindungsgemäße Verfahren vorzugweise sowohl eine Variation der Akkommodation, durch veränderbare Fokussierung sowie der Konvergenz durch vertikale Bewegung der Fixationsmarke erzielt werden.

Das Verfahren ermöglicht es, ein Gesamtbild bezogen auf die Querrichtung zum Verlauf der streifenförmigen Aufteilung mit einem Bildwinkel von mindestens 45°, vorzugsweise mindestens 60°, besonders vorzugsweise mindestens 65° des Fundus abzudecken, d. h. abzubilden.

Dadurch, dass die dritten sowie die vierten Purkinjereflexionen R3 und/oder R4 im Fundus scharf abgebildet werden, können diese als optische Bezugsposition zur Ermittlung der relativen Position der Detektionsoptik in Bezug auf die Blickrichtung des Auges herangezogen werden.

Die erfindungsgemäße Funduskamera umfasst zweckmäßigerweise ein Okular mit einer Feldblende, wodurch das Bildfeld weitwinkelig begrenzt werden kann. Hierbei ist vorteilhaft, ein kommerzielles Okular z. B. aus dem Bereich der Amateurastronomie oder Mikroskopie zu verwenden, welches den Vorteil einer optimal an das Auge angepassten Optik sowie geringer Kosten aufgrund der Massenmarktverfügbarkeit mit sich bringt.

Die schräge Ausrichtung des Displays zur Bildebene B5 ermöglicht ohne Einsatz von beweglichen Teilen (solid state device) eine Veränderung der Fokussierung, indem die auf dem Display angegebenen Fixationsmarke (Pixel) horizontal bewegt wird, wodurch sich der Abstand der Fixationsmarke auf dem Display zur Projektionslinse des Displays verändert. Diese variable Fokussierungsmöglichkeit ermöglicht eine Anpassung der Akkommodation des Patienten unter Berücksichtigung eventueller Fehlsichtigkeiten.

Der Einsatz der Farben Infrarot, Grün und Ultraviolett (IRGUV) zur Beleuchtung des Fundus bietet den Vorteil, dass auch bei Verwendung einer Schwarzweiß-Digitalkamera Farbbilder gewonnen werden können. Dadurch, dass eine Schwarzweiß-Digitalkamera gegenüber einer Farbdigitalkamera eine höhere Lichtsensitivität aufweist, kann bei Einsatz einer Schwarzweiß-Digitalkamera die Intensität der Beleuchtung und daraus resultierend die Patientenbelastung reduziert werden. Zur Erzeugung von Farbbildern werden sequenziell Schwarzweiß-Bilder aufgenommen und jeweils mit unterschiedlichen Farben, Infrarot, Grün bzw. Ultraviolett beleuchtet. Die drei hierdurch erzeugten Bilder werden den drei Primärfarben Rot, Grün und Blau zugeordnet und durch additive Farbmischung am Computer ein Farbbild rekonstruiert. Diese Methode bedingt eine geringere Belastung des Patienten aufgrund der reduzierten Lichteinwirkung sowie durch eine Reduzierung bzw. Vermeidung störender Nachbilder. Durch die geringere Beleuchtungsintensität wird ferner eine Reduzierung es Einflusses des Pupillenschließreflexes erreicht, wodurch bedingt durch eine größere Pupille bessere Bilder erzielt werden können. Daneben ist ein Infrarot-Vorschau-Modus zur Ausrichtung und Zentrierung der Funduskamera in einfacher Weise möglich.

Gemäß einer Ausgestaltung der Erfindung ist vorgesehen, dass mehrere Graustufenbilder des Fundus sequentiell aufgenommen werden, wobei jeweils nur eine Einzel-LED der Lichtquelle aktiv ist.

Gemäß einer weiteren Ausgestaltung der Erfindung ist vorgesehen, dass je Emissionswellenlänge der Lichtquelle ein oder mehrere Dunkelreferenzbilder aufgenommen werden, indem das Auge durch einen Lichtabsorber ersetzt ist.

Gemäß einer weiteren Ausgestaltung der Erfindung ist vorgesehen, dass je Emissionswellenlänge der Lichtquelle ein oder mehrere Hellreferenzbilder aufgenommen werden, wobei das Auge durch einen diffusen Reflektor ersetzt ist, welcher als Weißreferenz dient.

Gemäß einer weiteren Ausgestaltung der Erfindung ist vorgesehen, dass Artefakte in den Fundusbildern und Bildrauschen zumindest teilweise entfernt werden, indem von den Fundusbildern jeweils ein Dunkelreferenzbild oder die Mittelung mehrerer Dunkelreferenzbilder jeweils subtrahiert wird.

Gemäß einer weiteren Ausgestaltung der Erfindung ist vorgesehen, dass ein Farbabgleich und eine Reduktion des Bildrauschens durch eine pixelweise Division eines Fundusbildes durch ein Hellreferenzbild oder durch die Mittelung mehrerer Hellreferenzbilder erreicht werden kann.

Gemäß einer weiteren Ausgestaltung der Erfindung ist vorgesehen, dass mehrere Graustufenbilder des Fundus, welche jeweils bei unterschiedlichen Emissionswellenlängen der Lichtquelle aufgenommen wurden, zu einem Farbbild, Falschfarbenbild oder Multispektralbild kombiniert werden, wobei vorzugsweise eine relative Verschiebung und/oder Rotation und/oder Skalierung der Graustufenbilder vorher vorgenommen wird, so dass im kombinierten Bild die abgebildeten Strukturen des Fundus zur Deckung gebracht werden.

Gemäß einer weiteren Ausgestaltung der Erfindung ist vorgesehen, dass mehrere Graustufenbilder, Farbbilder, Falschfarbenbilder oder Multispektralbilder verrechnet werden, so dass die Auflösung erhöht und/oder der Rauschanteil verringert und/oder der sichtbare Bereich der Fundus vergrößert wird.

Gemäß einer weiteren Ausgestaltung der Erfindung ist vorgesehen, dass aus mehreren Fundusbildern, welche nach einem oder mehreren der oben genannten Verfahren erzeugt wurden, eine Videosequenz erzeugt wird. Dies hat den Vorteil, dass das erfindungsgemäße Verfahren auch mit Untersuchungsverfahren (z. B. Fluoreszenzangiographie), vorzugsweise zeitgleich, kombiniert werden kann.

Das, auch nebengeordnet beanspruchte, Patientenführungsmodul ermöglicht eine Führung des Auges des Patienten bei gleichzeitiger Möglichkeit einer Veränderung der Akkommodation und Ausgleich von Fehlsichtigkeiten durch Fokussierung ohne bewegliche Teile in der apparativen Konzeption.

### Beschreibung der Erfindung anhand von Ausführungsbeispielen

In den Zeichnungsfiguren werden beispielhafte Ausgestaltungen der vorliegenden Erfindung im Detail näher erläutert.

Es zeigen:
- Fig. 1: eine Darstellung eines ersten Beispiels einer erfindungsgemäßen Funduskamera;
- Fig. 2: eine Darstellung der streifenförmigen Aufteilung der Bildfläche in beleuchtete sowie unbeleuchtete Bereiche bei der Funduskamera nach Fig. 1;
- Fig. 3: eine Aufteilung der Apertur (Pupille) bei der Funduskamera nach Fig. 1;
- Fig. 4: eine fotografische Aufnahme der Bildfläche gemäß Fig. 2;
- Fig. 5: eine stark vereinfachte Darstellung einer mit der erfindungsgemäßen Funduskamera erstellten fotographischen Aufnahme eines vergrößerten Bereichs der Netzhaut;
- Fig. 6: eine zweckmäßige Ausgestaltung des erfindungemäßen Patientenführungsmoduls für den Einsatz bei einer Funduskamera mit Ermöglichung einer variablen Fokussierung;
- Fig. 7: ein Beispiel einer Funduskamera mit binokularer Patientenführung gemäß der vorliegenden Erfindung sowie
- Fig. 8: eine Darstellung eines weiteren Beispiels einer erfindungsgemäßen Funduskamera.

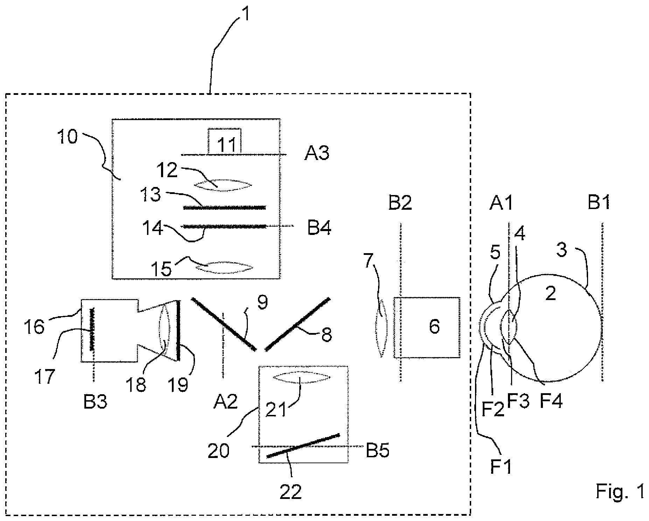

Die Bezugsziffer 1 in Fig. 1 bezeichnet die erfindungsgemäße Funduskamera in ihrer Gesamtheit. Die Funduskamera 1 dient dazu, den Augenhintergrund eines Auges 2 zu untersuchen und fotografische Aufnahmen davon anzufertigen.

Bei der Beleuchtung des Fundus des Auges 2 treten aufnahmebedingte Bildstörungen (Artefakte) im Fundusbild auf, welche durch die Rückreflexion der Beleuchtungsquelle entstehen. Diese Artefakte werden auch als "erste bis vierte Purkinjereflexionen" bezeichnet. Die Entstehungsorte dieser Purkinjereflexionen R1-R4 sind mit F1-F4 in Fig. 1 gekennzeichnet. Sie treten an unterschiedlichen Stellen im Auge auf. Die erste Purkinjereflexion R1 tritt an der Vorderseite der Cornea 5 (Hornhaut) auf und ist mit Abstand am stärksten. Die zweite Purkinjereflexion R2 tritt an der Rückseite der Cornea 5 auf. Die dritten und vierten Purkinjereflexionen R3 sowie R4 treten durch Fokussierung bzw. Zerstreuung an der Vorderseite bzw. Rückseite der Linse 4 des Auges 2 auf.

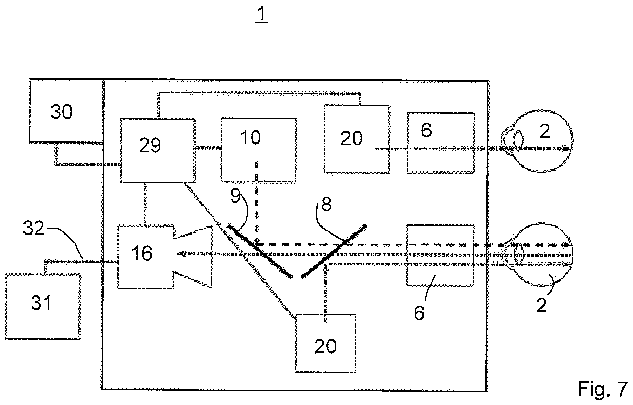

Die Funduskamera 1 umfasst ein Beleuchtungsmodul 10 mit einer annähernd punktförmigen Lichtquelle 11, vorzugsweise in Form eines LED-Moduls. Das LED-Modul umfasst drei Emitter, mit den Farben Infrarot, Grün und Ultraviolett. Der Einsatz der Farben Infrarot, Grün und Ultraviolett ermöglicht die Verwendung einer Schwarzweiß-Kamera, welche im Vergleich zu einer Farbkamera eine höhere Lichtsensitivität aufweist, wodurch die Intensität der Beleuchtung und damit die Patientenbelastung reduziert werden kann. Das Ein- und Ausschalten der einzelnen LED-Emitter wird durch einen Mikrocontroller 29 und einen Computer 31 gesteuert (vgl. auch Fig. 7).

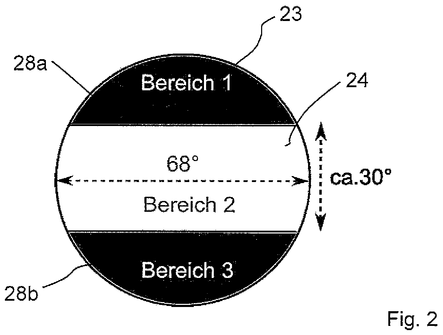

Die Kollimatoren 12 und 15 bilden die annähernd punktförmige Lichtquelle 11 auf den Spiegel 9 ab. Ein Polarisationsfilter 13 dient zusammen mit einem weiteren, vor der digitalen Kamera 16 positionierten Polarisationsfilter 19 dazu, Reflexionen in einem Okular 6 und im Auge 2 abzuschwächen. Ferner ist eine Spaltblende 14 vorgesehen, mittels welcher ein streifenförmiger Beleuchtungsbereich erzeugt und auf dem Fundus 3 abgebildet wird (vgl. Bereich 2 in Fig. 2).

Als Einrichtung zur Erzeugung digitaler, fotografischer Aufnahmen des Fundus 3 wird eine digitale Kamera 16, vorzugsweise eine digitale Schwarzweiß-Kamera verwendet. Die digitale Kamera 16 hat beispielsweise eine Auflösung von zwei Megapixeln. Die digitale Kamera 16 umfasst ein lichtempfindliches elektronisches Bauelement 17 zur Erfassung zweidimensionaler elektronischer Bilder. Des Weiteren umfasst die digitale Kamera 16 ein Objektiv 18. In die digitale Kamera 16 kann eine Autofokuseinheit zum Ausgleich von sphärischen Fehlsichtigkeiten integriert sein. Die Funduskamera 1 umfasst des Weiteren das Okular 6, dessen Anpassung an den Aufbau der Funduskamera 1 durch eine Feldlinse 7 vorgenommen ist.
Mit 20 ist ein Patientenführungsmodul gekennzeichnet. Dieses dient dazu, eine geführte Änderung der Blickrichtung des Patienten mittels einer vom Patientenführungsmodul 20 erzeugten Fixationsmarke zu veranlassen. Hierdurch wird erreicht, dass ausgewählte Bereiche des Fundus abgebildet, d. h. detektiert werden können. Teilaufhahmen können so zu einer umfassenden Gesamtdarstellung des Augenhintergrunds zusammengeführt werden.

Die Bezugsziffer 22 bezeichnet ein Display, vorzugsweise ein sogenanntes OLED-Display, zur Erzeugung der elektronischen Fixationsmarke. Die dem Display 22 vorgeschaltete Display-Projektionsoptik 21 bildet das Display 22 mit der darauf erzeugten Fixationsmarke auf der Zwischenbildebene B2 im Okular 6 ab. Der Strahlteiler 8 dient dazu, das Patientenführungsmodul 20 in den Aufbau zu integrieren.

Im optischen Aufbau der Funduskamera 1 gibt es die Bildebenen B1 bis B5, welche durch Linsen ineinander abgebildet werden. Die Bildebene B1 liegt auf dem Fundus 3 des Patienten. Der Patient "sieht" dieses Bild. Die Bildebene B2 stellt ein Zwischenbild im Inneren des Okulars 6 dar, dessen Bildabschnitt durch die Feldblende in horizontaler Richtung begrenzt wird im Beispiel der Fig. 2 auf einen kreisförmige Bildausschnitt entsprechend einem Sehwinkel von 68°. Die Bildebene B3 stellt das Bild dar, welches auf dem lichtempfindlichen elektrischen Bauelement 17 der digitalen Kamera 16 liegt. Dieses Bild der Bildebene B3 "sieht" die Kamera. Die Bildebene B4 bildet den homogen durchleuchteten Spalt der Spaltblende 14 ab. Die Bildebene B5 ist die Bildebene des Displays 22, in dem die Fixationsmarke erzeugt wird.

Befindet sich ein Objekt in einer der Bildebenen B1 bis B5, so wird es in allen anderen Bildebenen abgebildet. Von der Bildebene B1 zur Bildebene B3 wird der Fundus auf dem lichtempfindlichen, elektronischen Bauelement 17 der digitalen Kamera 16 abgebildet. Von der Bildebene B4 zur Bildebene B1 wird der holnogen durchleuchtete Spalt der Spaltblende 14 auf dem Fundus 3 bei gleichmäßiger Beleuchtung derselben abgebildet. Von der Bildebene B5 auf die Bildebene B1 wird über die Display-Projektionsoptik 21 sowie den Strahlteiler 8 eine vom Display 22 angezeigte Fixationsmarke auf dem Fundus 3 abgebildet.

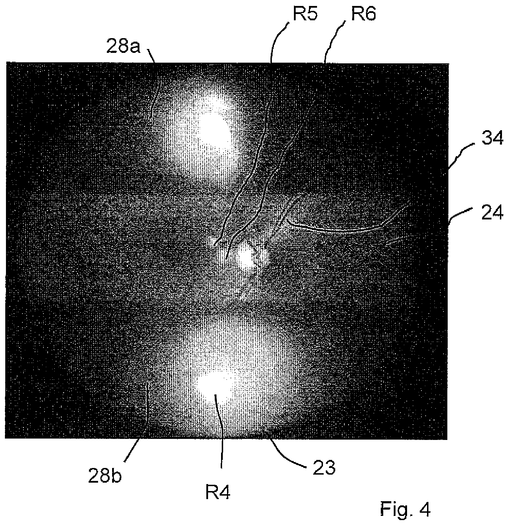

Fig. 2 zeigt ein Beispiel der streifenförmigen Aufteilung der Bildfläche 23 in der Bildebene B1. In der Mitte der Bildfläche 23 befindet sich der beleuchtete Streifen 24 als Abbildung der Spaltblende 14 des Beleuchtungsmoduls 10. An der Ober- und Unterseite des beleuchteten Streifens 24 befindet sich ein unbeleuchteter Bereich 28a bzw. 28b. Der beleuchtete Streifen 24 entspricht in dem gezeigten Beispiel somit einem Bildwinkel in horizontaler Richtung von ca. 68° sowie einem Bildwinkel in vertikaler Richtung von z. B. 30°. Durch die streifenförmige Festlegung des Bildfeldes 23 können die Artefakte, welche durch die Purkinjereflexionen R3 und R4 verursacht werden in den unbeleuchteten Bereich 28a bzw. 28b verlagert werden. Hierdurch wird vermieden, dass die Purkinjereflexionen R3 und R4 den beleuchteten Bereich des Fundus beeinträchtigen. Die Bildfläche 23 wird durch den vollen kreisförmigen Bereich innerhalb der Feldblende des Okulars 6 festgelegt. Der beleuchtete Streifen 24 kann beispielsweise in der Funduskamera 1 hinsichtlich seiner Hauptrichtung feststehend sein, d.h. er unterliegt keiner Translationsbewegung. Zweckmäßigerweise kann aber eine Spaltbreitenveränderung an der Spaltblende 14 vorgesehen sein. Wie aus der fotografischen Darstellung gemäß Fig. 4 hervorgeht, befinden sich durch Purkinjereflexionen verursachte Artefakte, z. B. die dritte und/oder vierte Purkinjereflexion R3 bzw. R4 in dem Bereich 28a und/oder 28b. Der beleuchtete Streifen 24 ist hingegen somit frei von Reflexionen der annähernd punktförmigen Lichtquelle an der Augenlinse. Verbleibende Reflexionen R5 und R6 haben ihren Ursprung im Okular 6.

Eine weitere Gruppe von Ebenen, welche in dem Aufbau ineinander abgebildet werden, sind die Aperturebenen A1 bis A3. Bei der Aperturebene A1 handelt es sich um die Pupille des Auges. In der Aperturebene A2 wird die Trennung der Strahlengänge der Beobachtung und Beleuchtung durch den Spiegel 9 vorgenommen. Die Aperturebene A3 ist die Ebene der Lichtquelle 11 mit dem LED-Modul. Die Gestaltung der Aperturebenen A1 bis A3 ermöglicht in erster Linie das Ausblenden der Artefakte der ersten und zweiten Purkinjereflexion R1 bzw. R2, d. h. die Purkinjereflexionen R1 bzw. R2 werden ausgelöscht noch bevor sie auf dem Bildsensor 17 abgebildet werden oder zumindest zum Teil eine Reduktion der Intensität der durch die Purkinjereflexionen verursachten Artefakte.

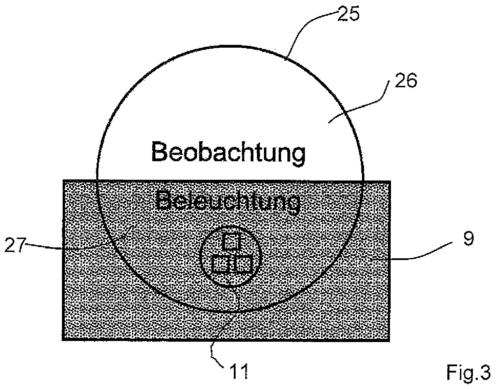

Der Spiegel 9 nimmt eine Trennung der Strahlenlänge der Beobachtung und Beleuchtung vor, wie dies in Fig. 3 wiedergegeben ist. Bezugsziffer 25 in Fig. 3 kennzeichnet die Pupille, also die natürliche Öffnung, durch die Licht in das Innere des Auges 2 fallen kann. Der Spiegel 9 unterteilt die Fläche der Pupille 25 in einen kreissegmentförmigen Beobachtungsbereich 26 sowie einen kreissegmentförmigen Beleuchtungsbereich 27. Im Beleuchtungsbereich 27 liegt das Abbild der annähernd punktförmigen Lichtquelle 11. In der Fig. 3 sind die beiden Bereiche 26 und 27 zum Beispiel halbkreisförmig ausgebildet. Ebenso gut können diese beiden Bereiche auch kreisabschnittförmig ausgebildet sein. Die Aperturteilung der Pupille 25 gemäß Fig. 3 ist vorzugsweise konstant. Der diesbezügliche aparative Aufbau ist ortsfest. Lediglich die Linse des Objektivs 18 der digitalen Kamera 16 kann zur Scharfstellung verschoben werden. Daraus resultiert ein besonders einfacher, kostengünstiger Aufbau der Funduskamera 1.

Mittels der Aperturteilung gemäß Fig. 3 werden die erste sowie zweite Purkinjereflexion R1 sowie R2 beseitigt oder zumindest die Intensität der durch die Purkinjereflexionen verursachten Artefakte reduziert. Die Aperturebene A1 liegt auf der Oberfläche der Linse 4 im Bereich der dritten Purkinjereflexion R3, sodass sich eine scharfe Abbildung der Purkinjereflexionen R3 und/oder R4 und damit eine Eingrenzung der hierdurch beeinträchtigten Bildfläche ergibt, wie dies aus Fig. 4 ersichtlich ist. Durch die Ausrichtung des Aufbaus derart, dass die dritten und/oder vierten Purkinjereflexionen R3 und R4 in die ungenutzten Bereiche 28a sowie 28b gemäß Fig. 2 abgebildet werden, lassen sich die vorgenannten Reflexionen nahezu vollständig aus dem Bild beseitigen. Da eine annähernd punktformige Lichtquelle 11 verwendet wird und die aus der Verwendung der annähernd punktförmigen Lichtquelle entstehenden Purkinjereflexionen R3 und/oder R4 in den Bereichen 28a sowie 28b vergleichsweise scharf abgebildet vorliegen, kann zudem durch die Auswertung der Position der Purkinjereflexion R3 und/oder R4 im Bild zusätzlich auf die relative Position der Funduskamera zum Auge geschlossen werden. Daraus resultiert die Möglichkeit einer, insbesondere automatischen, Zentrierung der Funduskamera 1 auf das Auge 2.

Die erfindungsgemäße Funduskamera 1 ermöglicht es, den Flächenbereich des zu untersuchenden Fundus 3 dadurch erheblich zu vergrößern, dass das Auge 2 bei dem vorbeschriebenen ortsfesten, d. h. statischen Aufbau des Beleuchtungsstrahlengangs sowie des Detektionsstrahlengangs geführt bewegt wird, wodurch sich der Bereich des Fundus 3 des Patienten unter dem beleuchteten Streifen 24 der Bildfläche 23 verschiebt und sich die dortige Fläche in der Bildfläche 23 verändert. Hierdurch ist es möglich, vertikale Bereiche des Fundus 3 von weit mehr als 30° aufzuzeichnen. Hierdurch wird mit einfachen Mitteln die Möglichkeit geschaffen, umfangreiche Flächenbereiche des Fundus 3 einer Untersuchung zuzuführen, wobei auf die Verabreichung von pupillenerweiterten Mitteln (Mydriatikum) verzichtet werden kann, wodurch sich ein erhöhter Patientenkomfort einstellt und keine aufwändigen, mit einer Verstellmechanik versehenen Aufbauten verwendet werden müssen.

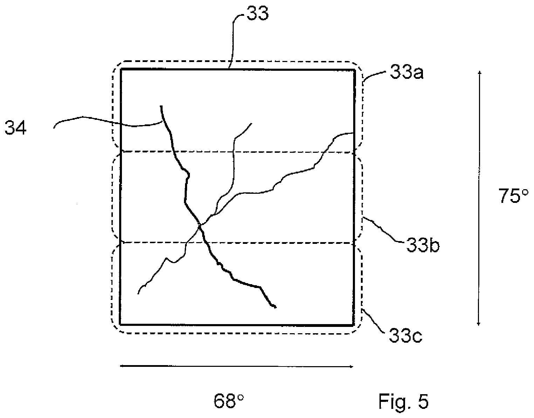

Das für eine solche Patentenführung vorgesehene Patientenführungsmodul 20 (vgl. Fig. 1) ermöglicht die Erzeugung einer Fixationsmarke (Leuchtmaske) auf den Fundus 3 in der Bildebene B1. Durch Veränderung der Fixationsmarke wird der Patient veranlasst, die Augenstellung im Vergleich zur digitalen Kamera 16 entsprechend der Positionsänderung der Fixationsmarke zu verändern, beispielsweise indem er vertikal nach oben oder nach unten der Fixationsmarke folgt und sich dadurch der zu untersuchende Bereich des Fundus 3 unter dem beleuchten Streifen 24 verändert. In Kombination mit einer Ansteuerung der Patientenführung kann die Blickrichtung des Patienten so gesteuert werden, dass unterschiedliche Bereiche der Netzhaut abgebildet werden und diese durch Kombination zu einem größeren Gesamtbild zusammengefügt (Stitching) werden. Hierdurch kann auch in vertikaler Richtung ein Bereich von 68° und mehr erreicht werden. Aus der Fig. 5 ist in stark vereinfachter schematischer Darstellungsweise eine entsprechende mittels Patientenführung erstellte Aufnahme 33 in einem erweiterten Winkelbereich des Fundus 3 skizziert. Die Aufnahme 33 ist aus drei Einzelaufnahmen 33a-33c aufgebaut, die zu der Aufnahme 33 zusammengesetzt wurden. Auf der Aufnahme ersichtliche Blutgefäße sind mit 34 gekennzeichnet.

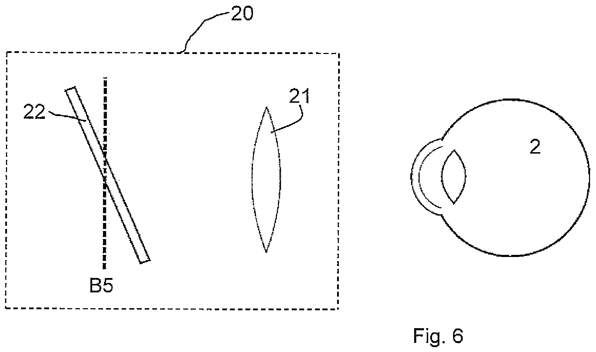

Die Fig. 6 zeigt das Prinzip des Patientenführungsmoduls 20 zur Ermöglichung einer Fokussierung, d. h. Anpassung der Akkommodation des Patienten ohne bewegbare Teile. Dies wird erreicht durch eine Schrägstellung des Displays 22 relativ zur Bildebene B5. Die Variation der Fixationsrichtung wird durch eine vertikale Bewegung, d. h. eine Bewegung senkrecht zur Zeichenebene der Fig. 6 des als Fixationsmarke dienenden Lichtpunkts bzw. Pixels erreicht. Die Variation der Akkommodation und der Ausgleich von sphärischen Fehlsichtigkeiten erfolgt demgegenüber durch eine horizontale Bewegung, d.h. durch eine Bewegung parallel zur Zeichenebene der Fig. 6. Durch die Schräglage des Displays 22 gegenüber der Bildebene B5 verändert sich bei horizontaler Bewegung des Bildpunkts (Verschiebung des Bildpunkts parallel zur Zeichenebene von Fig. 6) der Abstand zur Display-Projektionsoptik 21. Ein entsprechend in Fig. 6 dargestelltes Patientenführungsmodul 20 ist vorzugsweise für jedes Auge vorgesehen, um die Akkommodation variieren und eventuelle Fehlsichtigkeiten ausgleichen zu können.

Die in Fig. 7 dargestellte Ausgestaltung einer Funduskamera 1 mit binokularer Patientenführung umfasst ein Patientenführungsmodul 20 der beschriebenen Art für jedes Auge 2 sowie einen Mikrocontroller 29, welcher die Bewegung der Fixationsmarken in dem dem jeweiligen Auge 2 zugehörigen Patientenführungsmodul 20 steuert. Ein Detektionsstrahlengang zwischen dem Auge 2 und der digitalen Kamera 16 ist lediglich bei einem Auge 2 vorgesehen. Die digitale Kamera steht über eine geeignete Datenleitung 32, zum Beispiel über eine Ethernet-Schnittstelle mit einem Computer 31 in Verbindung. Durch eine Positioniereinheit 30 kann die Funduskamera 1 optimal auf die Augen ausgerichtet werden. Vorzugsweise ist die Positioniereinheit motorisiert und durch den Mikrocontroller 29 und den Computer 31 automatisch oder manuell steuerbar. Als Grundlage für eine automatische Steuerung kann die Position der Artefakte der Purkinjereflexionen R3 und/oder bzw. R4 im Bildfeld 23 dienen.

Der gesamte Aufbau der Funduskamera in Fig. 7 kann um eine zwischen den beiden Augen verlaufende (nicht dargestellte) Achse um 180° geschwenkt werden, sodass statt des einen Auges auch das andere Auge untersucht werden kann.

Bei einäugigen Patienten kommt nur der abbildende Teil der Anordnung zum Einsatz.

Der Mikrocontroller 29 empfängt zudem Steuerkommandos vom Computer 31, um die LEDs der Lichtquelle 11 in der richtigen Reihenfolge ein- und auszuschalten. Gleichzeitig wird vom Computer 31 die digitale Kamera 16 ausgelöst, sodass Beleuchtung und Belichtung synchron ablaufen. Die digitale Kamera 16 verfügt über Ein- und Ausgänge um eine Kommunikation und die Übermittlung von Steuerbefehlen vom Computer 31 zum Mikrocontroller 29 zu ermöglichen sowie Beleuchtung und Belichtung zu synchronisieren. Alternativ kann der Computer auch direkt mit dem Mikrocontroller 29 verbunden sein. In die digitale Kamera 16 kann eine Autofokuseinheit integriert sein, um sphärische Fehlsichtigkeiten ausgleichen zu können.

Mit Hilfe der Software auf dem Computer werden sequentiell gewonnene Grauwertbilder zu einem einzigen Farbbild kombiniert. Hierbei ist es erforderlich, einen eventuell vorhandenen Versatz der Bilder zu erkennen (Registrierung), diesen entsprechend auszugleichen und dann die Bilder zu einem Farbbild zu kombinieren. Der Computer ist mit einem Nutzerinterface ausgestattet, welches die Bedienung und Steuerung der Funduskamera sowie die Anzeige der Fundusaufnahmen ermöglicht.

Unter fotografischer Beobachtung und/oder Dokumentation im Sinne dieser Erfindung fallen sowohl statische fotografische Aufnahmen als auch Film- bzw. Video aufnahmen.

Im einfachen Betriebsmodus der Funduskamera bildet diese, vgl. Fig. 1, nur den Fundus 3 mit der Retina ab. In diesem Betriebsmodus der Funduskamera liegt die Bildebene B1, wie in Fig. 1 angegeben, auf dem Fundus 3 bzw. der Retina des Auges 2 und die Aperturebene A1 in, oder in der Nähe oder innerhalb der Augenlinse 4.

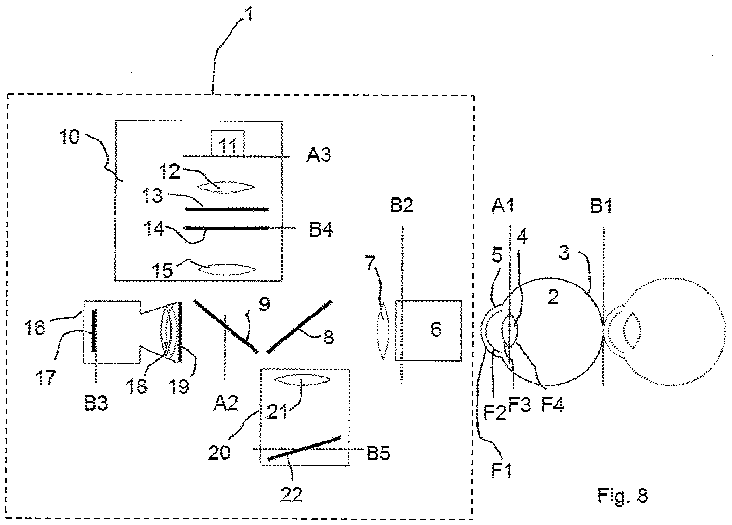

In einem erweiterten Betriebsmodus kann der Abstand der Funduskamera 1 zum Auge 2 erhöht und/oder die Fokuseinstellung des Objektivs 18 variiert werden, wie dies in Fig. 8 vereinfacht dargestellt ist (vgl. die punktiert dargestellten Positionen des Auges 2 sowie des Objektivs 18). Vorzugsweise können beide Vorgänge aufeinander abgestimmt stattfinden.

Alternativ oder zusätzlich zu den vorgenannten Maßnahmen kann auch die Ausrichtung der Funduskamera 1 zum Auge 2 verändert werden. Unter Ausrichtung ist zu verstehen (wobei dies in Fig. 8 nicht abgebildet ist): Die Winkelstellung der optischen Achse der Funduskamera 1 zur optischen Achse des Auges 2, die Winkelposition der optischen Achse der Funduskamera 1 zur optischen Achse des Auges 2 und/oder eine Rotation der Funduskamera 1 um deren optische Achse. Unter optischer Achse der Funduskamera 1 ist insbesondere die optische Achse des Okulars 6 zu verstehen.

Dadurch kann die Bildebene B1 in beliebige Bereiche im Auge nach vorne bis zu dem Bereich F1 verschoben werden. Dadurch können, wenn B1 in dem entsprechenden Bereich zu liegen kommt, z.B. Bereiche des Glaskörpers, dies ist in Fig. 1 bzw. Fig. 8 der Bereich zwischen F4 und dem Fundus 3, die Augenlinse 4, die Vorderkammer, die Iris, dies ist in Fig. 1 der Bereich zwischen F2 und F3, und/oder die Cornea, dies ist in Fig. 1 der Bereich zwischen F1 und F2, abgebildet werden. Dies ermöglicht es, den Grad der Untersuchungsmöglichkeiten des Auges 2 noch zu steigern.

Die Aperturebene A1 verschiebt sich in diesem erweiterten Betriebsmodus ebenfalls nach vorne im Auge 2, bis zu einer Position außerhalb des Auges 2, d.h. sie verschiebt sich ausgehend von einer Position in oder in der Nähe der Linse 4 in Richtung Cornea 5 und schließlich in einen Bereich zwischen Auge 2 und Funduskamera 1.

### BEZUGSZEICHENLISTE

- 1: Funduskamera
- 2: Auge
- 3: Fundus (Augenhintergrund)
- 4: Linse
- 5: Cornea (Hornhaut)
- 6: Okular
- 7: Feldlinse
- 8: Strahlteiler
- 9: Spiegel
- 10: Beleuchtungsmodul
- 11: Lichtquelle
- 12: Kollimator
- 13: Polarisationsfilter
- 14: Spaltblende
- 15: Kollimator
- 16: Digitale Kamera
- 17: Bildsensor
- 18: Objektiv
- 19: Polarisationsfilter
- 20: Patientenführungsmodul
- 21: Display-Projektionsoptik
- 22: Display
- 23: Bildfläche
- 24: beleuchteter Streifen
- 25: Pupille
- 26: Beobachtungsbereich
- 27: Beleuchtungsbereich
- 28: unbeleuchteter Bereich
- 29: Microcontroller
- 30: Positioniereinheit
- 31: Computer
- 32: Datenleitung
- 33: Aufnahme
- 34: Blutgefäß

- A1-A3: Aperturebenen
- B1-B5: Bildebenen
- F1-F4: Entstehungsorte der ersten bis vierten Purkinjereflexionen
- R1-R4: Erste bis vierte Purkinjerelexion
- R5-R6: Reflexionen des Okulars

## Patentansprüche

1. Verfahren zur fotografischen Beobachtung und/oder Dokumentation des Fundus eines Auges, welches folgende Verfahrensschritte umfasst:
Erzeugung eines Beleuchtungsstrahlengangs mittels eines Beleuchtungsmoduls zur Beleuchtung mindestens einer Teilfläche des Fundus unter Einsatz einer zumindest annähernd punktförmigen oder zumindest flächenmäßig begrenzten Beleuchtungsquelle,
Erzeugung einer Bildebene (B3) im Beleuchtungsstrahlengang für die Erstellung fotografischer Aufnahmen,
Auftrennung der Pupillenquerschnittsfläche in einen detektierbaren Beobachtungsbereich sowie einen nicht detektierbaren Beleuchtungsbereich,
Erzeugung einer Bildfläche (23), die das Abbild des Fundus auf der Bildebene (B3) darstellt,
streifenförmige Aufteilung der Bildfläche (23) im Beleuchtungsstrahlengang in einen beleuchteten sowie unbeleuchtete Bereiche,
Abbildung mindestens einer Purkinjereflexion auf der Bildfläche (23), **dadurch gekennzeichnet, dass**
der Beleuchtungsstrahlengang relativ zum Gesamtaufbau der Funduskamera ortsfest ist
und sich die mindestens eine Purkinjereflexion in den unbeleuchteten Bereichen der Bildfläche (23) befindet.

2. Verfahren nach Anspruch 1, **dadurch gekennzeichnet, dass** der detektierbare Beobachtungsbereich sowie der nicht detektierbare Beleuchtungsbereich der Pupillenquerschnittsfläche eine halbkreisförmige oder kreisabschnittsförmige Ausprägung besitzt.

3. Verfahren nach Anspruch 1 oder 2, **dadurch gekennzeichnet, dass**
die Aperturebene A1 von der Pupille nach hinten ins Auge hinein verlegt wird, vorzugsweise in eine Ebene an der Vorderseite der Linse des Auges oder zumindest in eine Ebene im Bereich der Linse des Auges.

4. Verfahren nach mindestens einem der vorhergehenden Ansprüche, **dadurch gekennzeichnet, dass**
die dritten und/oder vierten Purkinjereflexionen zumindest im Wesentlichen auf der Bildebene (B3) scharf abgebildet werden.

5. Verfahren nach mindestens einem der vorhergehenden Ansprüche, **dadurch gekennzeichnet, dass**
dem beleuchteten Bereich der Bildfläche (23) ein Bildwinkel in Querrichtung zum Streifen von maximal 5°-30°, vorzugsweise von 20° und/oder ein Bildwinkel in Längsrichtung zum Streifen von 30°-80°, vorzugsweise von 68° entspricht.

6. Verfahren nach mindestens einem der vorhergehenden Ansprüche, **dadurch gekennzeichnet, dass**
der Detektionsstrahlengang relativ zum Gesamtaufbau der Funduskamera ortsfest ist.

7. Verfahren nach mindestens einem der vorhergehenden Ansprüche, **dadurch gekennzeichnet, dass**
die im Beleuchtungsstrahlengang sowie Detektionsstrahlengang zu untersuchende Fläche des Fundus durch Veränderung der Blickrichtung des Auges des Patienten relativ zur Ausrichtung des Beleuchtungsstrahlengangs und/oder Detektionsstrahlengang variiert wird, und/oder
die durch Veränderung der Blickrichtung des Auges des Patienten unterschiedlichen Bereiche des Fundus gesondert detektiert und zu einem Gesamtbild zusammengefügt werden, und/oder
ein Patientenführungsmodul vorgesehen ist, mittels dem die Blickrichtung des Patienten mittels einer Fixationsmarke im zu untersuchenden Auge oder im anderen Auge geführt wird, sodass durch Veränderung der Blickrichtung außerhalb der Detektion liegende Flächenbereiche des Fundus in den Bereich der Detektion verschoben werden.

8. Verfahren nach mindestens einem der vorhergehenden Ansprüche, **dadurch gekennzeichnet, dass**
die Positionen und/oder Formen der dritten und/oder vierten Purkinjereflexion im Fundusbild zur Ermittlung der räumlichen Position der Detektionsoptik relativ zur Linse des Auges verwendet werden.

9. Funduskamera zur fotografischen Beobachtung, Dokumentation und/oder Diagnose der Netzhaut eines Auges (2), insbesondere zur Durchführung des Verfahrens gemäß mindestens einem der vorstehenden Ansprüche umfassend
ein Beleuchtungsmodul (10) mit vorzugsweise annähernd punktförmiger oder zumindest flächenmäßig begrenzter Lichtquelle (11) zur Erzeugung eines Beleuchtungsstrahlenganges,
eine Spaltblende (14) zur Begrenzung des Beleuchtungsstrahlenganges auf einen beleuchteten Streifen (24) des Fundus (3) neben unbeleuchteten Bereichen (28a, 28b),
eine digitale Kamera (16),
einem Detektionsstrahlengang zwischen der digitalen Kamera (16) und des Fundus (3), **dadurch gekennzeichnet, dass**
Mittel zur Unterteilung des Detektionsstrahlengangs in einen detektierbaren Beobachtungsbereich (26) sowie einen nicht detektierbaren Beleuchtungsbereich (27) vorgesehen sind, wobei
mindestens eine Purkinjereflexion jeweils im unbeleuchteten Bereich (28a, 28b) abgebildet ist und
der Beleuchtungsstrahlengang relativ zum Gesamtaufbau der Funduskamera ortsfest ist.

10. Funduskamera nach Anspruch 9, **dadurch gekennzeichnet, dass** der Detektionsstrahlengang relativ zum Gesamtaufbau der Funduskamera ortsfest ist.

11. Funduskamera nach Anspruch 9 oder 10, **dadurch gekennzeichnet, dass**
der detektierbare Beobachtungsbereich (26) sowie der nichtdetektierbare Beleuchtungsbereich (27) eine halbkreisförmige oder kreisabschnittsförmige Ausprägung aufweisen.

12. Funduskamera nach Anspruch 9 bis 11, **dadurch gekennzeichnet, dass**
zur Unterteilung des Detektionsstrahlengangs ein Spiegel (9) vorgesehen ist und die erste und zweite Purkinjereflexion (R1, R2) im nicht detektierbaren Beleuchtungsbereich (27) liegen, und/oder
die dritte und/oder vierte Purkinjereflexion (R3, R4) auf dem Bildsensor (17) abgebildet werden.

13. Funduskamera nach einem der Ansprüche 9 bis 12, **dadurch gekennzeichnet, dass**
die Aperturebene (A1) in einem Bereich zwischen Vorderseite der Hornhaut (5) und Rückseite der Linse (4) des Auges (2) liegt, vorzugsweise in der Mitte der Linse.

14. Funduskamera nach einem der Ansprüche 9 bis 13, **dadurch gekennzeichnet, dass**
ein Patientenführungsmodul (20) vorgesehen ist, mittels welchem durch gezielte Führung der Blickrichtung des Patienten der zu untersuchende Bereich des Fundus zur Position des Beleuchtungsstrahlengangs sowie Detektionsstrahlengangs veränderbar ist, und
das Patientenführungsmodul (20) ein Display (22) aufweist, das Display (22) zur Erzeugung einer Fixationsmarke für das Auge (2) dient und die Fixationsmarke des Displays (22) auf den Fundus des Auges (2) abgebildet wird.

15. Funduskamera nach Anspruch 14, **dadurch gekennzeichnet, dass**
das Patientenführungsmodul (20) eine Bildebene B5 sowie eine Display-Projektionsoptik (21) aufweist und das Display (22) relativ zur Bildebene B5 eine schräge Ausrichtung hat.

16. Funduskamera nach einem der Ansprüche 14 oder 15, **dadurch gekennzeichnet, dass**
eine Variation der Blickrichtung durch relative Bewegung der Fixationsmarke parallel zur und/oder eine Variation der Scharfstellung durch eine Bewegung der Fixationsmarke senkrecht zur Schnittlinie zwischen Display (22) und Brennebene (B5) erfolgt.

17. Funduskamera nach einem der Ansprüche 14 bis 16, **dadurch gekennzeichnet, dass**
in jedem Auge (2) durch ein zugehöriges Patientenführungsmodul (20) eine Fixatonsmarke erzeugt wird und die Bewegung der Fixationsmarken über einen Microcontroller (29) für beide Augen (2) gesteuert wird.

18. Patientenführungsmodul zur Führung der Blickrichtung des Auges des Patienten zur Erzeugung einer Fixationsmarke, umfassend
ein Display (22) zur Erzeugung einer Fixationsmarke auf dem Display (22),
insbesondere für den Einsatz in dem Verfahren gemäß einem der Ansprüche 1 bis 8 und/oder der Funduskamera gemäß einem der Ansprüche 9 bis 17, einer Display-Projektionsoptik (21), mittels welcher die Fixationsmarke über optische Elemente auf den Fundus des Auges (2) projizierbar ist und eine Bildebene B5 im Display (22) angeordnet ist, **dadurch gekennzeichnet, dass**
das Display (22) die Bildebene B5 der Display-Projektionsoptik in spitzem Winkel durchsetzt, wodurch bei Variation der Position der Fixationsmarke parallel zur Schnittlinie vom Display (22) und der Bildebene B5 eine Änderung der Blickrichtung ohne Änderung der Fokussierung sowie bei der Änderung der relativen Lage der Fixationsmarke senkrecht zur Schnittlinie zusätzlich eine variable Fokussierung und damit ein Ausgleich von sphärischen Fehlsichtigkeiten und eine Steuerung der Akkommodation des Patienten durchführbar ist.

19. Funduskamera nach mindestens einem der vorhergehenden Ansprüche 9 bis 17, **dadurch gekennzeichnet, dass**, die Funduskamera (1) derart hergerichtet ist, dass der Abstand und/oder die Ausrichtung der Funduskamera (1) zum Auge (2) und/oder die Fokuseinstellung des Objektivs (18) variierbar ist.

## Claims

1. Method for photographic observation and/or documentation of the fundus of an eye, comprised of the following procedural steps:
generating an illumination beam by way of a lighting module in order to illuminate at least a partial area of the fundus, through the use of an at least approximately punctiform light source or one which is at least limited in area;
generating an image plane (B3) in the illumination beam to create photographic images;
separating the cross-section of the pupil into a detectable area of observation, as well as a non-detectable area of illumination;
generating an image surface (23) which represents the reflection of the fundus in the image plane (B3);
splitting the image surface (23) in the illumination beam into strips, thus creating an illuminated area, as well as unilluminated areas;
displaying at least one Purkinje reflection on the image surface (23),
**characterised in that**
the illumination path is stationary in relation to the overall structure of the fundus camera
and the Purkinje reflection, of which there is at least one, is located in the unilluminated areas of the image surface (23).

2. Method according to claim 1, **characterised in that** the detectable area of observation, as well as the non-detectable areas of illumination of the pupil cross-section, have the shape of a semi-circle or a circular segment.

3. Method according to claim 1 or 2, **characterised in that** the aperture plane A1 is moved back from the pupil into the eye, preferably to a plane on the front of the lens of the eye, or at least to a plane in the area of the lens of the eye.

4. Method according to at least one of the preceding claims, **characterised in that**
the third and/or fourth Purkinje reflections appear in focus on the image plane (B3) to at least a substantial degree.

5. Method according to at least one of the preceding claims, **characterised in that**
the illuminated area of the image surface (23) corresponds to a transverse angle of view in relation to the strip of a maximum of 5°-30°, preferably 20° and/or a longitudinal angle of view in relation to the strip of 30°-80°, preferably 68°.

6. Method according to at least one of the preceding claims, **characterised in that**
the detection beam is stationary in relation to the overall structure of the fundus camera.

7. Method according to at least one of the preceding claims, **characterised in that**
the surface of the fundus under examination in the illumination beam as well as the detection beam is varied by changing the viewing direction of the patient's eye in relation to the orientation of the illumination beam and/or detection beam, and/or
the different areas of the fundus, differing based on the change in viewing direction of the patient's eye, are detected separately and assembled into a complete image, and/or
a patient guidance module is provided for, by means of which the viewing direction of the patient is directed with a fixation mark in the eye under examination, or in the other eye, so that by changing the viewing direction, surface areas of the fundus outside of the area of detection may be shifted into the area of detection.

8. Method according to at least one of the preceding claims, **characterised in that**
the positions and/or forms of the third and/or fourth Purkinje reflection in the fundus image are used to determine the spatial position of the detection optics in relation to the eye.

9. Fundus camera for photographic observation, documentation and/or diagnosis of the retina of the eye (2), in particular by carrying out the method according to at least one of the preceding claims, comprising an illumination module (10) with a preferably nearly punctiform light source or one which is at least limited in area (11), used to generate an illumination beam,
a slit (14) to limit the illumination beam to one illuminated strip (24) of the fundus (3) aside from unilluminated areas (28a, 28b),
a digital camera (16),
a detection beam between the digital camera (16) and the fundus (3), **characterised in that**
means are provided for to divide the detection beam into a detectable area of observation (26), as well as a non-detectable area of illumination, whereby
at least one Purkinje reflection each is displayed in the unilluminated area (28a, 28b) and
the illumination beam is stationary in relation to the overall structure of the fundus camera.

10. Fundus camera according to claim 9, **characterised in that** the detection beam is stationary in relation to the overall structure of the fundus camera.

11. Fundus camera according to claim 9 or 10, **characterised in that** the detectable area of observation (26), as well as the non-detectable area of illumination (27) demonstrate the shape of a semi-circle or circular segment.

12. Fundus camera according to claims 9 to 11, **characterised in that** a mirror (9) is provided for to divide the detection beam, and the first and second Purkinje reflections (R1, R2) lie in the non-detectable area, and/or the third and/or fourth Purkinje reflections (R3, R4) are displayed on the image sensor (17).

13. Fundus camera according to one of claims 9 to 12, **characterised in that** the aperture plane (A1) lies in an area between the front of the cornea (5) and the back of the lens (4) of the eye (2), preferably in the middle of the lens.

14. Fundus camera according to one of claims 9 to 13, **characterised in that** a patient guidance module is provided for, by means of which the area of
the fundus under examination may be changed to correspond with the position of the illumination beam as well as the detection beam, by deliberately guiding the viewing angle of the patient, and
the patient guidance module (20) is equipped with a display (22), the display (22) serves to generate a fixation mark for the eye (2) and the fixation mark of the display (22) is displayed on the fundus of the eye (2).

15. Fundus camera according to claim 14, **characterised in that**
the patient guidance module (20) is equipped with an image plane B5, as well as an optical display projection system (21), and the display (22) is diagonally oriented in relation to the image plane B5.

16. Fundus camera according to one of claims 14 or 15, **characterised in that**
a variation of the viewing angle is achieved through relative movement of
the fixation mark parallel to the line of intersection between the display (22) and focal plane (B5) and/or a variation of the focus is achieved by moving the fixation mark perpendicular to the line of intersection between
the display (22) and focal plane (B5).

17. Fundus camera according to one of claims 14 to 16, **characterised in that**
a fixation mark is generated in each eye (2) with an accompanying patient guidance module (20) and the movement of the fixation mark is controlled for both eyes (2) with a microcontroller (29).

18. Patient guidance module to guide the viewing angle of the patient's eye in order to generate a fixation mark, comprising
a display (22) to generate a fixation mark on the display (22),
particularly for use in the method according to one of claims 9 to 17, an optical display projection system (21), by means of which the fixation mark may be projected through optical elements on to the fundus of the eye (2) and an image plane B5 is positioned in the display (22), **characterised in that**
the display (22) permeates the image plane B5 of the optical display projection system (21) at an acute angle, whereby, by varying the position of the fixation mark parallel to the line of intersection between the display (22) and image plane B5, the viewing angle may be modified, without changing the focus; and by modifying the relative position of the fixation mark perpendicular to the line of intersection, variable focussing is possible, and thus a compensation of spherical vision defects and control of the patient's accommodation.

19. Fundus camera according to at least one of the preceding claims 9 to 17, **characterised in that** the fundus camera (1) is arranged in a way that the distance and/or orientation of the fundus camera (1) in relation to the eye (2) and/or the focus adjustment of the lens (18) in variable.

## Revendications

1. Procédure d'observation photographique et/ou documentation du fond d'un oeil comprenant des étapes opérationnelles suivantes :
Production d'un faisceau lumineux par un module de formation d'images pour éclairer au moins une surface partielle du fond de l'oeil en utilisant une source lumineuse au moins approximativement sous forme de points ou du moins restreinte en superficie,
production d'un plan d'image (B3) dans le faisceau lumineux pour réaliser des photographies,
découpage de la section transversale de la pupille dans une zone d'observation détectable ainsi que dans une zone d'éclairage non détectable,
réalisation d'une surface d'image (23) représentant l'image du fond de l'oeil sur le plan d'image (B3),
découpage sous forme de bandes de la surface d'image (23) dans le faisceau lumineux dans une zone éclairée et dans une zone non éclairée,
projection d'au moins une réflexion Purkinje sur la surface d'image (23),
**caractérisée en ce que**
le faisceau lumineux est fixe par rapport à la structure générale du rétinographe
et qu'au moins une réflexion Purkinje se trouve dans les zones non éclairées de la surface d'image (23).

2. Procédure selon la revendication 1 **caractérisée en ce que** la zone d'observation détectable ainsi que la zone d'éclairage non détectable de la section transversale de la pupille comprennent une empreinte semi-circulaire ou en forme de segment de cercle.

3. Procédure selon les revendications 1 ou 2 **caractérisée en ce que** le plan d'ouverture A1 de la pupille est décalé vers l'arrière de l'oeil, de préférence dans un plan situé sur le devant du cristallin de l'oeil ou du moins dans la zone du cristallin de l'oeil.

4. Procédure selon au moins l'une des revendications précédentes **caractérisée en ce que**
les images des troisièmes et/ou quatrièmes réflexions Purkinje offrent dans l'ensemble une représentation nette sur le plan d'image (B3).

5. Procédure selon au moins l'une des revendications précédentes **caractérisée en ce**
**qu'**à la zone éclairée du plan de l'image (23) correspond un angle de vision dans la direction transversale à la bande de 5°-30° maximum, de préférence de 20°, et/ou un angle de vision dans la direction longitudinale à la bande de 30°-80°, de préférence de 68°.

6. Procédure selon au moins l'une des revendications précédentes **caractérisée en ce que**
le faisceau de détection est fixe par rapport à la structure générale du rétinographe.

7. Procédure selon au moins l'une des revendications précédentes **caractérisée en ce que**
la surface du fond de l'oeil à examiner dans le faisceau lumineux ainsi que dans le faisceau de détection varie selon le changement de la direction du regard du patient par rapport au positionnement du faisceau lumineux et/ou du faisceau de détection, et/ou
les différentes parties du fond de l'oeil, résultant du changement de la direction du regard du patient, sont détectées séparément et regroupées pour former une image globale, et/ou
un module de guidage du patient est prévu, à l'aide duquel la direction du regard du patient est dirigée par une marque de fixation dans l'oeil à examiner ou dans l'autre oeil, afin qu'en cas de changement de la direction du regard les parties du fond de l'oeil situées à l'extérieur de la zone de détection soient déplacées dans la zone de détection.

8. Procédure selon au moins l'une des revendications précédentes **caractérisée en ce que**
les positions et/ou les formes de la troisième et/ou de la quatrième réflexion Purkinje sur l'image du fond de l'oeil sont utilisées pour détecter la position dans l'espace de l'optique de détection par rapport au cristallin de l'oeil.

9. Le rétinographe destiné à l'observation photographique, la documentation et/ou le diagnostic de la rétine d'un oeil (2) et, particulièrement, l'application de la procédure selon au moins l'une des revendications précédentes comprenant
un module d'éclairage (10) avec une source lumineuse (11), de préférence approximativement sous forme de points ou du moins restreinte en superficie pour produire un faisceau lumineux,
un diaphragme à fente (14) pour circonscrire le faisceau lumineux sur une bande éclairée (24) du fond de l'oeil (3) à côté des zones non éclairées (28a, 28b),
un appareil photographique numérique (16),
un faisceau de détection entre l'appareil photographique numérique (16) et le fond de l'oeil (3), **caractérisé en ce que**
des moyens de découpage du faisceau de détection dans une zone d'observation détectable (26) et dans une zone d'éclairage non détectable (27) sont prévus, étant donné
qu'au moins une réflexion Purkinje est à chaque fois projetée dans la zone non éclairée (28a, 28b) et
que le faisceau lumineux est fixe par rapport à la structure générale du rétinographe.

10. Rétinographe selon la revendication 9 **caractérisé en ce que** le faisceau de détection est fixe par rapport à la structure générale du rétinographe.

11. Rétinographe selon les revendications 9 ou 10 **caractérisé en ce que** la zone d'observation détectable (26) ainsi que la zone d'éclairage non détectable (27) ont une empreinte semi-circulaire ou en forme de segment de cercle.

12. Rétinographe selon les revendications 9 à 11 **caractérisé en ce qu'**un miroir (9) est prévu pour segmenter le faisceau de détection, que les première et deuxième réflexions Purkinje (R1, R2) se trouvent dans la zone d'éclairage non détectable (27), et/ou que les troisième et/ou quatrième réflexions Purkinje (R3, R4) sont projetées sur le capteur d'images (17).

13. Rétinographe selon l'une des revendications 9 à 12 **caractérisé en ce que** le plan d'ouverture (A1) se trouve entre la face antérieure de la cornée (5) et la face arrière de la lentille (4) de l'oeil (2), de préférence au milieu de la lentille.

14. Rétinographe selon l'une des revendications 9 à 13 **caractérisé en ce qu'**un module de guidage du patient (20) est prévu, à l'aide duquel la partie du fond de l'oeil à examiner peut être modifiée selon le positionnement du faisceau lumineux ainsi que du faisceau de détection par le guidage ciblé de la direction du regard du patient, et
que le module de guidage du patient (20) comprend un écran de visualisation (22), l'écran de visualisation (22) sert à générer une marque de fixation pour l'oeil (2) et la marque de fixation de l'écran de visualisation (22) est projetée sur le fond de l'oeil (2).

15. Rétinographe selon la revendication 14 **caractérisé en ce qu'**un module de guidage du patient (20) comprend un plan d'image B5 ainsi qu'un système optique de projection de l'écran de visualisation (21) et que l'écran de visualisation (22) a une orientation oblique par rapport au plan d'image B5.

16. Rétinographe selon l'une des revendications 14 ou 15 **caractérisé en ce qu'**une variation de la direction du regard, due à un mouvement relatif de la marque de fixation, se produit parallèlement à la ligne de coupe entre l'écran de visualisation (22) et le plan focal (B5) et/ou une variation de la mise au point causée par le mouvement de la marque de fixation a lieu perpendiculairement à la ligne de coupe entre l'écran de visualisation (22) et le plan focal (B5).

17. Rétinographe selon l'une des revendications 14 à 16 **caractérisé en ce qu'**une marque de fixation est générée dans chaque oeil (2) par un module de guidage du patient correspondant (20) et que le mouvement des marques de fixation est dirigé par un microcontrôleur (29) pour chaque oeil (2).

18. Module de guidage du patient pour orienter la direction du regard du patient afin de générer une marque de fixation comprenant
un écran de visualisation (22) pour générer une marque de fixation sur l'écran de visualisation (22),
en particulier pour l'emploi dans la procédure selon l'une des revendications 1 à 8 et/ou le rétinographe selon l'une des revendications 9 à 17, une optique de projection de l'écran de visualisation (21) à l'aide de laquelle la marque de fixation peut être projetée via des éléments optiques sur le fond de l'oeil (2) et un plan d'image B5 positionné sur l'écran de visualisation (22), **caractérisé en ce que**
l'écran de visualisation (22) pénètre sous un angle aigu le plan d'image B5 du système optique de projection de l'écran de visualisation, ce qui permet, en cas de variation de la position de la marque de fixation parallèlement à la ligne de coupe de l'écran de visualisation (22) et du plan d'image B5, de réaliser un changement de direction du regard sans modification de la focalisation, tout comme, en cas de changement de la position relative de la marque de fixation perpendiculairement à la ligne de coupe, une focalisation variable est possible permettant ainsi la compensation de défauts de vision sphériques et l'accommodation du patient.

19. Rétinographe selon au moins l'une des revendications 9 à 17 **caractérisé en ce que** le rétinographe (1) est positionné de telle manière que la distance et/ou l'orientation du rétinographe (1) par rapport à l'oeil (2) et/ou l'ajustement focal de l'objectif (18) est variable.
